Europäisches Patentamt

European Patent Office

Office européen des brevets

(11) Veröffentlichungsnummer: **0 261 566**
**A2**

(12) **EUROPÄISCHE PATENTANMELDUNG**

(21) Anmeldenummer: 87113511.7

(22) Anmeldetag: 16.09.87

(51) Int. Cl.⁴: **C12N 9/24** , C12N 9/16 ,
C12N 9/88

(30) Priorität: 20.09.86 DE 3632086

(43) Veröffentlichungstag der Anmeldung:
30.03.88 Patentblatt 88/13

(84) Benannte Vertragsstaaten:
BE CH DE FR GB IT LI NL SE

(71) Anmelder: **Verein der Zuckerindustrie**
**Am Hofgarten 8**
**D-5300 Bonn(DE)**

(72) Erfinder: **Buchholz, Klaus, Dr.,Dipl.-Chem.**
**Rueninger Weg 44**
**D-3300 Braunschweig(DE)**
Erfinder: **Arntz, Hans-Juegen, Dipl.-Chem.**
**Brunnenstr. 22**
**D- Meine-2, Ortsteil Grassel(DE)**

(74) Vertreter: **Döring, Rudolf, Dr.-Ing. et al**
**Patentanwälte Dr.-Ing. R. Döring Dipl.-Phys.**
**Dr. J. Fricke Jasperallee 1a**
**D-3300 Braunschweig(DE)**

(54) **Verfahren zur Behandlung von polysaccharidhaltigen Substanzen.**

(57) Die Erfindung sieht ein Verfahren zur Gewinnung polysaccharidspaltender Enzyme durch Umsetzung von polysaccharidhaltigen Substanzen, wie Nebenprodukten der Nahrungs-und Zuckerindustrie durch Fermentation mit Mischkulturen hydrolysierender Bakterien vor, und zwar durch folgende Verfahrensmaßnahmen:

a) Konzentration der polysaccharidhaltigen Substrate auf wenigstens 1 g Trockensubstanz je Liter Wasser,

b) Einstellung und Aufrechterhaltung eines pH-Wertes zwischen 5 und 7,5,

c) Einstellung der Temperatur auf Werte zwischen 20° und 40°C,

d) Verweilzeit der Flüssigphase auf mindestens 12 Stunden bis zu einigen Tagen,

e) Abtrennung der Feststoffe und Konzentration der Lösung.

EP 0 261 566 A2

## Verfahren zur Behandlung und Umsetzung von polysaccharidhaltigen Substraten

Die Erfindung betrifft ein Verfahren zur Behandlung und Umsetzung von polysaccharidhaltigen Substraten, wie Nebenprodukten der Nahrungsmittel-und Zuckerindustrie, durch Fermentation mit Mischkulturen hydrolysierender Baktieren.

Es sind Verfahren vorgenannter Art bekannt, um aus Nebenprodukten der Nahrungsmittel-und Zuckerindustrie Biogas zu gewinnen (K. Buchholz, H.J. Arntz, A. Pellegrini, E. Stoppok, Zuckerind. 111 (1986) Nr. 9.

Unter dem Begriff der Nebenprodukte der Nahrungsmittel-und Zuckerindustrie sollen auch die dabei entstehenden Abfallprodukte erfaßt werden, welche polysaccharid haltige Substrate enthalten, wie beispielsweise polysaccharidhaltige Feststoffe, wie extrahierte Rübenschnitzel oder Rübenabfälle, nämlich Blatteile, Rübenbruchstücke und dgl.

Bei dem bekannten Verfahren erfolgt die Umsetzung anaerob mittels der genannten Mischkulturen hydrolysierender Bakterien, die z.B. aus Bodenproben oder Proben aus einem anaeroben Verfahren, wie z.B. bei der Abwasserreinigung, gewonnen werden.

Aufgabe der Erfindung ist es, das einleitend genannte Verfahren so weiterzubilden, daß eine Umsetzung der polysaccharidhaltigen Substrate erreicht und hydrolytische Enzyme dabei gewonnen werden.

Bei der als Hydrolyse bezeichneten eingangs genannten Fermentation hat sich überraschenderweise herausgestellt, daß unter geeigneten Bedingungen hohe Konzentrationen an extrazellulären Pektinasen, Hemizellulasen und Zellulasen in der Fermentationsflüssigkeit vorliegen. Ausgehend von diesen Erkenntnissen sieht die Erfindung vor, daß polysaccharidspaltende Enzyme im Zuge des einleitend genannten Verfahrens gewonnen werden durch

a) Einstellung der Konzentration der polysaccharidhaltigen Substrate auf wenigstens 1 g Trockensubstanz je Liter Wasser,

b) Einstellung und Aufrechterhaltung eines pH-Wertes zwischen 5 und 7,5,

c) Einstellung der Temperatur auf Werte zwischen 20° und 40°C,

d) Verweilzeiten der Flüssigphase auf mindestens 12 Stunden bis zu einigen Tagen,

e) Abtrennung der Feststoffe und Konzentrierung der Lösung.

Es ist bekannt, daß polysaccharidspaltende Enzyme in der Technik eine zunehmende Bedeutung erlangen, insbesondere in der Nahrungsmittelindustrie. Besondere Anwendungsmöglichkeiten sind in der Abwasser-und Abfallbehandlung zu sehen. Die bisherige Gewinnung der vorgenannten Enzyme bereitet erhebliche Schwierigkeiten und ist mit sehr hohen Kosten verbunden. Die hohen Kosten ergeben sich zum einen durch die bisher notwendige Verwendung teurer Substrate und durch die Notwendigkeit, die Fermentation steril durchzuführen, so daß hohe Investitionskosten für die Fermentationseinrichtungen notwendig sind. Außerdem erfordern die in der Regel aerob durchgeführten Fermentationen einen hohen Energieaufwand zur Belüftung des Fermenters.

Demgegenüber werden erfindungsgemäß für die Gewinnung der polysaccharidspaltenden Enzyme Rohstoffe verwendet, die in großen Mengen anfallen und in einer wahlweise steril oder unsteril durchgeführten anaeroben Fermentation mit den genannten Mischkulturen der hydrolysierenden Bakterien der Fermentation unterworfen werden können.

Dabei kann die Konzentration der polysaccharidhaltigen Substrate in weiten Grenzen variieren je nach Art der Fermentation. Im Falle der Fermentation eines Rohrreaktors kann die Konzentration der polysaccharidhaltigen Substrate so groß sein, daß eine pastenförmige Masse entsteht, welche durch den Rohrreaktor hindurchbewegt wird.

Zweckmäßig und insbesondere für die Verwendung in Rührfermentern ist es jedoch, wenn die Konzentration der polysaccharidhaltigen Substrate auf Werte zwischen 5 und 20g Trockensubstanz pro Liter Wasser eingestellt wird.

Der Einstellung und Aufrechterhaltung des pH-Wertes während der Fermentation kommt eine besondere Bedeutung zu, da die zu gewinnenden Enzyme ihre Hauptaktivität in dem pH-Bereich zwischen 5 und 7,5 haben. Besonders günstig ist es, wenn der pH-Wert zwischen 6 und 7 eingestellt wird. Diese pH-Wert-Einstellung gilt sowohl für das kontinuierliche als auch für das diskontinuierliche Verfahren bzw. für die kontinuierliche oder diskontinuierliche Fermentation.

Auch die Einstellung der Verfahrenstemperatur während der Fermentation ist von erheblicher Bedeutung. Als besonders günstig wurde der Temperaturbereich zwischen 30 und 37°C festgestellt.

Die Verweilzeiten der Flüssigphase kann in Abhängigkeit von dem gewünschten Aktivitätsspektrum in relativ weiten Grenzen variieen, wobei jedoch die Untergrenze bei etwa 12 Stunden liegt und die Obergrenze nach den bisherigen Erfahrungen bis zu 5 Tagen betragen kann.

Günstig ist es, wenn die Verweilzeit der Flüssigphase in Abhängigkeit von der Enzymaktivität auf Werte eingestellt wird, bei denen eine merkbare Abflachung der Aktivitätssteigerung feststellbar ist.

Die Abtrennung der Feststoffe kann in bekannter Weise, z.B. durch Zentrifugieren oder Filtration, erfolgen. Dabei kann zunächst eine Grobfiltration erfolgen und die durchtretende Flüssigkeit nachfolgend von suspendierten feinen Feststoffen in einer nachfolgenden Filtration befreit werden. Die Enzyme können anschließend in einem Ultrafiltrationsschritt um das 5-bis 100fache konzentriert werden, so daß man ein hochakti ves Konzentrat erhält. Ein derartiges Konzentrat kann praktisch ohne Aktivitätsverlust bei 4°C über mehrere Monate gelagert werden.

Zur Erzeugung von Starterkulturen für des obengenannte Verfahren sieht die Erfindung vor, daß polysaccharidhaltige Bodenproben oder Proben aus anaeroben Verfahren mit einem Gehalt von 0,01 bis 1 g Trockenmasse an hydrolysierenden Bakterien je Liter Flüssigkeit mit darin vorgesehenen polysaccharidhaltigen Substraten in einer Konzentration von 1 bis 50 g pro Liter Flüssigkeit bei einer Temperatur von 20 bis 40°C sowie einem pH-Wert von 5 bis 7,5 so lange vorfermentiert werden, bis der CSB-Wert des Substrates auf 20 bis 50% des Ausgangswertes vermindert ist, ehe durch Abtrennung der groben Feststoffe die Kulturflüssigkeit gewonnen wird.

Beispiel 1:

Es wird die halbkontinuierliche Produktion der hydrolytischen Enzyme in einem 6l-Fermenter im "fed-batch-Verfahren" beschrieben.

In einem Rührreaktor mit einem Blattrührer von 70 mm Durchmesser und einer Drehzahl von 200 U/min mit Umwurf werden 6 l Kulturflüssigkeit eingefüllt und einmal täglich 100 g Rübenpreßschnitzel mit 23 Gew.% TS zugegeben.

Als Inoculum (Starterkultur)dient ein an das Substrat angepaßter Versäuerungsschlamm. Dieser wird dadurch gewonnen, daß Bakterienschlamm aus einer anaeroben Abwasserreinigung in einer Konzentration von 0,5 g Trockenmasse je Liter Flüssigkeit mit Rübenpreß schnitzeln bei einem Feststoffgehalt von 20 g Trockensubstanz je Liter bei 35 bis 40°C 48 Stunden fermentiert wird. Nach dem Abtrennen des groben Feststoffes enthält der Überstand eine Bakterientrockensubstanz von 1 bis 2 g je Liter Suspension.

Die Fermentation wird durch Zugabe von 1 Liter Inoculum gestartet und bei 35°C unter Luftabschluß durchgeführt.

Im weiteren Verlauf der Gärung wird 24 Stunden nach Zugabe des Substrates 1 Liter Suspension dem Fermenter entnommen. Der Feststoff wird abzentrifugiert und in den Fermenter zurückgeführt und das fehlende Nutzvolumen durch Wasser ergänzt.

Der Überstand, der die extrazellulären Enzyme enthält, wird mit Hilfe einer Querstrom-Ultra-Filtrationseinheit mit einer Ausschlußgrenze von 10 000 Dalton konzentriert.

Folgende Enzymaktivitäten lassen sich nachweisen:
Endo-Pektathydrolasen
Pektatylasen
Pektinesterasen
Endo-$\beta$-Glucanasen (CMCase)
Endo-Arabanhydrolasen
Galactanhydrolasen.

Beispiel 2:

Diskontinuierliche Herstellung hydrolytischer Enzyme.

In einem 150 l-Rührfermenter mit Blattrührer, 200 mm Durchmesser und einer Drehzahl von 120 U/min werden 140 l Leitungswasser und 10 kg feuchte Rübenpreßschnit zel gegeben und mit Inoculum, wie im Beispiel 1 erläutert, angeimpft. Die Fermentation erfolgt bei 35°C und einem pH-Wert von 6,5, der automatisch durch Zugabe von Natronlauge korrigiert wird. Nach einer Lag-Phase von ca. 12 Stunden beginnen die Mikroorganismen, die gewünschten Enzyme in das Medium auszuscheiden. Das Maximum der Enzymaktivitäten ist bereits nach ca. 20 Stunden erreicht.

Aus dem Fermenter wird die Fermentationsflüssigkeit abgezogen, grob filtriert sowie nachfolgend mit Hilfe einer Querstrom-Mikrofiltrationseinheit mit einer Auschlußgrenze von 0,2 μm von Bakterien und Feststoff befreit, gesammelt und über eine Cross-flow-Ultrafiltrationseinheit mit einer Ausschlußgrenze von 10 000 Daltron konzentriert.

Das Konzentrat enthält folgende Aktivitäten:
Endo-Pektinhydrolasen
Pektatlyasen        3,2 n Kat/ml Kulturflüssigkeit
Endo-Arabanhydrolasen        300 mg/l Arabinoseanhydrid
Endo-Galactanhydrolasen        60 - 130 mg/l Galactoseanhydrid
Endo-$\beta$-Glucanasen (CMCase).

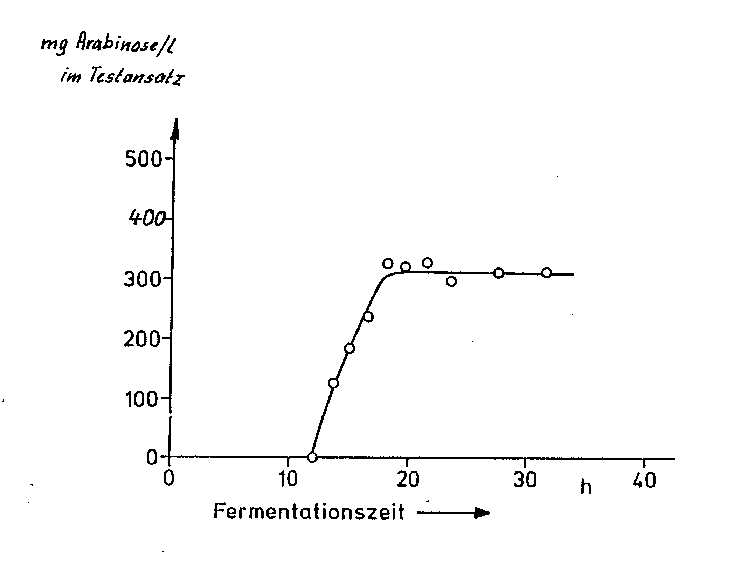

In der beigefügten Abbildung ist der Verlauf der Arabanhydrolaseaktivität als Funktion der Fermentationszeit gemäß Beispiel 2 wiedergegeben.

Die Aktivität der Endo-Araban-und Galactanhydrolasen wurde mit zerkleinertem Rübenmark als Substrat bestimmt. Ein Testansatz wird aus 2g Rübenmark, 20 ml Sörensen-Phosphatpuffer ph 6,5 und 20 ml gereinigter Fermentationsflüssigkeit zusammengestellt und 4 Stunden bei 40°C inkubiert. 500 µl aus dem Überstand werden membranfiltriert (0,45 µm) und mit einem im Handel erhältlichen Enzymgemisch, bestehend aus Zellulose und Pektinase, behandelt. Die entstehenden monomeren Zucker (Arabinose und Galactose) werden chromatographisch bestimmt und dienen als Maß für die genannten Enzymaktivitäten.

Die Bestimmung der Aktivität der Pektatlyasen erfolgte in Anlehnung an Rombouts, F.M. (1972): Occurence and properties of bacterial pectate lyases, Agric. Res. Rep. 779, Doctoral thesis, Wagening.

Der Nachweis der Endo-Pektinhydrolasen und Endo-$\beta$-Glucanasen erfolgte in Anlehnung an Rapp, P.; Reng,H., Hempel, D.-Chr., Wagner, F.: Cellulose degradation and monitoring of viscosity decrease in cultures of cellulomonas uda grown on printed newspaper, Biotechn. Bioeng. Vol. XXVI (1984) 1167 - 1175.

## Ansprüche

1. Verfahren zur Behandlung und Umsetzung von polysaccharidhaltigen Substraten, wie Nebenprodukten der Nahrungsmittel-und Zuckerindustrie, durch Fermentation mit Mischkulturen hydrolysierender Bakterien, **dadurch gekennzeichnet,** daß polysaccharidspaltende Enzyme gewonnen werden durch

a) Konzentration der polysaccharidhaltigen Substrate auf wenigstens 1 g Trockensubstanz je Liter Wasser,

b) Einstellung und Aufrechterhaltung eines pH-Wertes zwischen 5 und 7,5,

c) Einstellung der Temperatur auf Werte zwischen 20° und 40°C,

d) Verweilzeiten der Flüssigphase auf mindestens 12 Stunden bis zu einigen Tagen,

e) Abtrennung der Feststoffe und Konzentration der Lösung.

2. Verfahren nach Anspruch 1, **dadurch gekennzeichnet,** daß die Konzentration der polysaccharidhaltigen Substrate auf Werte zwischen 5 und 20 g Trockensubstanz pro Liter Wasser eingestellt wird.

3. Verfahren nach Anspruch 1 oder 2, **dadurch gekennzeichnet,** daß die Temperatur auf Werte von 30 bis 37°C eingestellt wird.

4. Verfahren nach einem der vorhergehenden Ansprüche, **dadurch gekennzeichnet,** daß die Verweilzeit der Flüssigphase in Abhängigkeit von der Enzymaktivität auf Werte eingestellt wird, bei denen eine merkbare Abflachung der Aktivitätssteigerung feststellbar ist.

5. Verfahren zur Erzeugung von Starterkulturen für das Verfahren nach einem der vorhergehenden Ansprüche, **dadurch gekennzeichnet,** daß polysaccharidhaltige Bodenproben oder Proben aus anaeroben Verfahren mit einem Gehalt von 0,01 bis 1 g Trockenmasse an hydrolysierenden Bakterien je Liter Flüssigkeit mit darin vorgesehenen polysaccharidhaltigen Substraten in einer Konzentration von 1 bis 50 g pro Liter Flüssigkeit bei einer Temperatur von 20 bis 40°C sowie einem pH-Wert von 4 bis 7,5 so lange und so oft vorfermentiert werden, bis der CSB-Wert des Substrates auf 20 bis 50 % des Ausgangswertes vermindert ist.